# EUROPÄISCHE PATENTSCHRIFT

(11) **EP 1 699 915 B1**
(45) Veröffentlichungstag und Bekanntmachung des Hinweises auf die Patenterteilung: **02.06.2010**
(21) Anmeldenummer: 04804424.2
(22) Anmeldetag: 30.12.2004
(51) Int. Cl.: A61K 38/18

(54) **VERWENDUNG DES ERYTHROPOIETINS ZUR REGENERATION VON LEBERGEWEBE**
USE OF ERYTHROPOIETIN FOR LIVER TISSUE REGENERATION
UTILISATION DE L'ERYTHROPOIETINE POUR LA REGENATION DE TISSU HEPATIQUE

(30) Priorität: 30.12.2003 DE 10361813; 30.12.2003 EP 03029961
(43) Veröffentlichungstag der Anmeldung: 13.09.2006
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2004/014839
(87) Internationale Veröffentlichungsnummer: WO 2005/063965

(56) Entgegenhaltungen:
- EP-A- 1 077 254
- EP-A1- 1 333 036
- WO-A-01/13936
- WO-A-99/21966
- WO-A-02/102432
- WO-A-2004/001023
- WO-A-2004/001023
- WO-A2-2004/012759
- DE-A1- 10 303 584
- US-A- 5 716 644
- US-A- 6 045 818
- US-A1- 2002 187 936
- US-A1- 2003 031 701
- US-A1- 2003 096 407
- US-B1- 6 479 064

## Beschreibung

Die vorliegende Erfindung betrifft die Verwendung von Erythropoietin zur Induktion des strukturellen Wachstums von Gewebe bei der Leberregeneration und nimmt die Prioritäten der deutschen Patentanmeldung 103 61 813.9-41 und der europäischen Patentanmeldung 03 029 961.4 in Anspruch.

In der Ontogenese werden Wachstumsfaktoren exprimiert, die grundlegende strukturelle und hinsichtlich der Zellzahl numerische Prozesse für den Aufbau eines Gewebes auslösen können. Im wachsenden und im adulten Organismus geht die Fähigkeit, strukturell und funktionell intakte Gewebe aufzubauen oderim Falle von Gewebeschädigungen - zu regenerieren, allerdings weitgehend verloren. Es wird vermutet, dass die verminderte Expression von Wachstumsfaktoren, die ihrerseits die Expression von für den Gewebeaufbau erforderlichen Proteinen kontrollieren, für diese Reduktion an Regenerationsfähigkeit verantwortlich ist.

Es ist jedoch zumindest von einige Organen bekannt, dass sie selbst im adulten Organismus eine Fähigkeit zur Selbst-Regeneration behalten, die durch Verletzungsprozesse induziert werden kann. So ist beispielsweise die Regenerationskapazität der Leber bereits seit der Antike bekannt. Fast alle anderen Organe können strukturelle Defekte nicht von sich aus entsprechend überbrücken um, das originale Gewebe wiederherzustellen.

Die Leber befindet sich im adulten Organismus in der Regel im ruhenden, das heißt nicht-proliferierenden Zustand, in dem das Organ eine komplexe Vielfalt verschiedener metabolischer Funktionen erfüllen muß. *In vivo* wird die Leber allerdings durch den Verlust von Zellmasse - z.B. durch eine Leberzellschädigung oder durch einen chirurgischen Eingriff - erneut zum Wachstum angeregt.

Proliferierendes Lebergewebe ersetzt die funktionellen und anatomischen Strukturen des Organs meist jedoch nicht in der gewünschten Weise, sondern führt in der Regel zu einer Vergrößerung und Hypertrophie des verbliebenen Lebergewebes, bis die ursprüngliche Leberzellmasse ersetzt ist. Die Intensität der Wachstumsantwort ist von dem Ausmaß des Gewebeverlustes abhängig. Der zeitliche Verlauf der Leberregeneration korreliert dabei umgekehrt proportional, das heißt kleine Leberzellverluste werden langsam ersetzt, große Leberzellverluste wesentlich rascher.

Der Ersatz der Organmasse durch Zellproliferation allein stellt daher keine ausreichende Basis für die Therapie eines Patienten mit erheblichem Organschaden dar. Es sind daher verschiedene Ansätze für die Induktion strukturellen Wachstums - d.h. für ein formschaffendes Wachstum - von Geweben bekannt, die jedoch allesamt noch nicht zu einem befriedigenden Erfolg geführt haben. Ein solches strukturelles Wachstum von Zellen wäre jedoch gerade für therapeutische oder biotechnologische Verfahren von erheblicher Bedeutung.

In der Vergangenheit wurde versucht, Wachstum von Zellen über die Gabe von Wachstumsfaktoren, wie z.B. "Epidermal Growth Facto" (EGF), "Vascular Endothelial Growth Factor" (VEGF) oder "Hepatocyte Growth Factor" (HGF) zu induzieren. Die Wirkung dieser Faktoren auf die Vermehrung primärer Zellen *in vitro* ist jedoch begrenzt. Ihr Einsatz *in vivo* ist dagegen aufgrund ihrer möglichen Nebenwirkungen - z.B. der Aktivierung von Onkogenen - nicht unproblematisch.

Ein anderer Ansatz basiert auf der Verwendung komplexer heterologer Gewebeextrakte, z.B. aus der Hypophyse oder dem Hypothalamus, zur Induktion der Zellvermehrung beispielsweise von kultivierten Hepatozyten (siehe z.B. US 6,008,047). Die Verwendung tierischer oder humaner Gewebeextrakte ist jedoch vor dem Hintergrund übertragbarer viraler Erkrankungen, wie z.B. BSE, Schweine- oder Schaf-Viren, im Laborbetrieb oder in der klinischen Anwendung problematisch und dokumentiert eher das mangelnde Wissen um die Prozesse, die an dem Aufbau komplexer Organstrukturen beteiligt sind sowie um die eigentlich relevanten Faktoren und deren Einsatz- und Wirkpotentiale. Zudem sind die Extrakte in ihrer Qualität schwer definierbar, da diese u.a. von der Quelle und deren Kultivierungsbedingungen abhängt.

Selbst die wenigen Erkenntnisse aus den klassischen Anwendungen der primären Gewebekulturen lassen sich allerdings nicht unmittelbar auf die Fragestellungen des *tissue engineering* übertragen. So geht das *tissue engineering* in der Regel idealerweise von patientenspezifischen, adulten Zellsystemen aus, die bereits weiter differenziert sind als fötale oder embryonale Zellen. Außerdem handelt es sich bei dem *tissue engineering* sowohl *in situ* als auch *in vitro* um Kokultursituationen, die in der klassischen Anwendung nicht berücksichtigt werden. Es wird dagegen viel eher sogar versucht, Kokulturen aus Endothelzellen, Makrophagen und Fibroblasten, wie sie in der Leber vorkommen, bei der Expansion der parenchymatösen Leberzellen zu vermeiden, da sie nicht erwünscht sind.

Aus der WO 02/092013 A2 ist es bekannt, einem Patienten zur Behandlung von Leberschäden eine therapeutisch effektive Menge von Wachstumshormon (Growth Hormone, GH) zu verabreichen, um damit das natürliche Regenerationsvermögen der Leber zu fördern. Danach hat GH die Wirkung, die Expression des Wachstumsfaktors Fox M1B bei Hepatozyten zur beschleunigten und so das Leberwachstum erneut zu initiieren.

Das Wachstumshormon hat jedoch eine sehr breite und daher unspezifische Wirkung auf das Wachstum von Geweben. Daher werden durch GH-Gabe auch ungewollte Nebenwirkungen oder Überreaktionen z.B. in Form der sogenannten Akromegalie, d. h. einer überschiessenden Verknöcherung mit pathologischen Knochenzuständen, erzeugt. Des weiteren wurde in der Zwischenzeit bekannt, dass Fox1M in Basalzellkarzinomen hochreguliert wird. Die Fox-Proteine spielen eine wichtige Rolle in der Regulation der Wachstumsgene bei der Vermehrung, Differenzierung und der Transformation, u.a. auch bei der Aktivierung von sogenannten SONIC HEDGEHOG (Shh) Signalwegen. Diese wiederum sind involviert in der Aktivierung von Basalzellkarzinomen in menschlicher Haut. So konnten Teh et al. zeigen (Cancer Research 2002, August 15; 62 (16): 4773-80), dass die Hochregulierung von FoxM1 in Basalzellkarzinomen eine der wesentlichen Initiations-Mechanismen ist, wodurch die SONIC HEDGEHOG-Signalwege mitogene Effekte in den basalen Kerotinozyten ausüben, wodurch es zu der Entwicklung des weit verbreiteten menschlichen Krebsgeschwürs kommt. Dieses tumorigene Potential von Fox1M Agonisten und die mangelnde Spezifität und ubiquitäre Präsenz in allen Geweben steht daher der Verabreichung von GH zur Förderung der Leberregeneration entgegen.

Die WO 2004/001023 lehrt unter anderem, dass EPO und TPO eine Induzierung struktureller Prozesse in vivo oder in vitro bewirken, in dem sie diese bei lokaler oder systemischer Gabe auslösen und terminieren können, insbesondere wenn über die induktive Wirkung eines Implantats in situ eine lokalspezifische Zellvermehrung initiiert wird.

Aufgabe der vorliegenden Erfindung ist es daher, ein Verfahren bereitzustellen, das das strukturelle Wachstum von Lebergewebe nach Leberresektionen in Folge von Tumoren, induziert und fördert. Dieses Wachstum sollte vorzugsweise zu einer im wesentlichen funktionellen und strukturellen Funktionsfähigkeit des betroffenen Gewebes führen.

Erfindungsgemäß wird diese Aufgabe gelöst durch den Einsatz von Erithropoietin, dessen Derivate oder Analoga bzw. Teilen für die strukturelle und funktionelle Leberregeneration. In einer besonders bevorzugten Ausführungsform wird Erythropoietin (EPO) oder ein Derivat, Teilen oder Analogon davon verabreicht.

Es hat sich überraschenderweise gezeigt, dass durch die Applikation des hämatopoietischen Wachstumsfaktores EPO nicht nur eine Vermehrung der Zellen, sondern auch ein strukturelles Wachstum einsetzt. Dieses Wachstum setzt insbesondere an zuvor traumatisierten Geweben ein. Das dadurch induzierte Wachstum führt *in vivo* zu einer Gewebe-Regeneration im eigentlichen Sinne, d.h. es findet nicht nur proliferatives Wachstum, sondern gerichtetes, differenziertes Wachstum zum Aufbau komplexer Strukturen statt.

Im Kern basiert die Verwendung des EPO für die Geweberegeneration im wesentlichen auf zwei bisher nicht bekannten Wirkungen des EPO, nämlich einerseits auf der Stimulation des strukturellen Wachstums in synchroner und koordinierter Form von verschiedener Zelltypen untereinander und miteinander (wie z.B. Fibroblasten, glatten Muskelzellen mit Endothelzellen im Gefäßbereich in Verbindung mit einer Neubildung der Architektur eines kompletten Gefäßes unter Berücksichtigung der extrazellulären Matrix (Kollagen, Elastin, Fibronektin, Entaktin)) und einer Komplettierung des eigentlichen parenchymatösen Gewebeverbandes. Dies beinhaltet z.B. die Ausbildung von Hepatozyten mit den verbundenen Kupffer Zellen, Pit- Ito- und Endothelzellen (sog. nicht parenchymale Zellen der Leber). Neben der Ausbildung eines tatsächlichen Gefäßbaumes und dessen Interkonnektion wird somit eine Geweberegeneration im Sinne der *restitutio ad integrum* induziert.

In der Leber führt dies zu einer Mischung aus sinusoidalen Kapillaren im Endstrombereich und vaskulären zu- und abführenden Gefäßen neben dem eigentlichen Parenchymverband der Hepatozyten in einer geordneten 3-D. Struktur.

Wie bereits ausgeführt, setzt die Wirkung der hämatopoietischen Wachstumsfaktors EPO insbesondere bei traumatisierten Geweben und Zellen ein. Dabei wird der Begriff des Traumas als Gegensatz zu dem Prozess der Histogenese (Gewebebildung) definiert. Es handelt sich demnach bei einem Trauma um einen Prozess, der der Histogenese als Gewebildungsprozess im Individualorgismus an den betreffenden Lokalisationen entgegenwirkt bzw. das Ergebnis der Histogenese zunichte macht. Das Trauma als Gewebeschädigung kann durch eine Vielzahl von Ereignissen ausgelöst werden, z.B. durch Verletzungen, Entzündungen oder durch Autoimmunerkrankungen mit Selbstbeschädigung). Diese Gewebeschädigungen oder -zerstörungen lösen wiederum eine Vielzahl von Reaktionen aus, so z.B. die Aktivierung von Makrophagen, Mastzellen und immunkompetenten Zellen, die chemotaktische, vasoaktive und wundheilungsfördernde Faktoren sezernieren, und dadurch systemische und regioselektive Mechanismen regulieren.

Die Vorteile der Verwendung des EPO, erstrecken sich auf die Regeneration von Geweben aller vier Grundgewebearten, nämlich des Bindegewebes, des Muskelgewebes, des Epithelgewebes und des Nervengewebes. Diese Gewebe leiten sich ontogenetisch entweder aus dem Mesoderm (Bindegewebe, Muskel, Endothel (als besondere Form des Epithels)), dem Endoderm (das den Gastrointestinaltrakt auskleidende Epithel) oder dem Ektoderm (Nervengewebe) ab. In der Vergangenheit wurde gezeigt, dass der EPO-Rezeptor sowohl auf Zellen meso- als auch endodermalen Ursprungs sowie auf neuronalen Zellen exprimiert wird.

In diesen Geweben führt die Verwendung von EPO zu einem ortsständigen Recruitment der gewebespezifischen Progenitorenpopulation (Stammzellen), der Migration der Zellen und der Differenzierung bzw. Transdifferenzierung der Zellen in Parenchym- und Strukturzellen. Während und vor dieser Gewebsbildung vermehren sich die Zellen durch die EPO Gabe.

Bei der Anwendung der erfindungsgemäßen Verwendung von EPO für die Leberregeneration kann eine erneute Vervollständigung des zuvor geschädigten Organs zu einem kompletten parenchymatösen Gewebeverband erreicht werden, einschließlich der Ausbildung von Hepatozyten mit Kupffer-Zellen, Pit-, Ito- und Endothelzellen. Mit der weiterführenden Ausbildung eines Gefäßbaums ist somit erfindungsgemäß die Geweberegeneration als *restitutio ad integrum* möglich.

Ein besonderer Vorteil der erfindungsgemäßen Verwendung von EPO liegt somit darin, daß nicht nur die Mikokapillarisation des regenerierenden Gewebes über eine Endothelaussprossung stimuliert wird, sondern auch die Parenchymregeneration und die Ausbildung der Wandstrukturen gefördert werden. Erst dies führt zu dem gewünschten Ergebnis des koordinierten dreidimensionalen Wachstums zum Aufbau eines funktionsfähigen Organs.

Somit basiert die erfindungsgemäße Verwendung auf einer EPO-Wirkung, die weit über die bisher bekannte EPO-Wirkung als angiogenetischer Faktor auf die Endothelzellenvermehrung hinausgeht (Journal of Nephrology 2002 15, 97 bis 103). Da mikrovaskuläre Strukturen wie Kapillaren und Sinusoide lediglich aus Endothelzellenauskleidung bestehen und keine eigene Wandstruktur aufweisen, konnte ausgehend von der angiogenetischen Wirkung des EPO bisher jedoch lediglich darüber spekuliert werden, ob EPO auch bei der Gefäßneubildung und Wundheilung eine gewisse Bedeutung zukommen könnte (Journal of Nephrology 2002 15,97 bis 103). Eine Substantiierung dieser Spekulationen blieb bislang aber aus.

Daher ist es vorliegend um so bedeutender, dass erstmals der Nachweis der Wirkung des EPO auf das synchronisierte und koordinierte Wachstum der Gefäße selbst, d.h. einschließlich der Ausbildung der Wandstrukturen und die Parenchymregeneration gelungen ist.

Ein weiterer Vorteil der Verwendung von EPO besteht darin, dass das strukturelle Wachstum nicht notwendigerweise eine vorgegebene organische oder anorganische dreidimensionale Struktur als Ausgangspunkt benötigt, sondern viel eher eine Organ(teil)struktur *de novo* schafft. Somit kann die Applikation des hämatopoietischen Wachstumsfaktors eine deutlich beschleunigte Selbst-Regeneration eines beschädigten Gewebes induzieren, was in der klinisch-therapeutischen Anwendung von großer Bedeutung ist.

In einer besonders vorteilhaften Ausführungsform wird der hämatopoietische Wachstumsfaktor eingesetzt, um die Regeneration eines Gewebes zu induzieren, das traumatisch geschädigte Bereiche aufweist. In diesen Gewebeabschnitten wird damit nicht nur ein Verschluß der Wunde durch die Ausbildung eines Granulationsgewebes mit einsetzender Angiogenese angeregt, sondern die Neubildung der gewebespezifischen dreidimensionalen Struktur aus extrazellulärer Matrix z.B. aus Kollagen, Elastin, Fibronektin oder Etnaktin.

Erfindungsgemäß werden als hämatopoietische Wachstumsfaktoren EPO bzw. dessen Teile, Derivate oder Analoga eingesetzt. Es eignen sich auch deren mimetische Peptide (siehe unten).

Bei den hämatopoietischen Wachstumsfaktoren handelt es sich um linksseitige Vierfachhelixbündelproteine mit einer Orientierung rauf - rauf - runter - runter mit zwei überreichenden Schleifen, die die ersten beiden und die letzten beiden Helixstrukturen miteinander verbinden (Livnah O. et al., Science 1999, 283 (5404): 987-90 und Ultsch M.H. et al, Blood 1995, 86 (2): 540-7. Die jeweiligen RBD Domainen (rezeptorbindenden Domänen) besitzten eine ausgeprägte Homologie mit EPO. Thrombopoietin, Erythropoietin und Wachstumshormon binden an den MPL-Rezeptorkomplex. In der Publikation von Youssoufianh et al. in Blood 1993, 819 2223 bis 36, Structure function and activation of the Erythropoietin receptor) wird eine produktive Dimerisation beschrieben. Erythropoietin und Thrombopoietin, mimetische Peptide (EMP und DMP) und weiterhin nicht-peptidische kleine Moleküle sind ebenso funktionell, wenn auch auf einer niedrigeren molaren Basis (Publikationen: Wrighton NC et al. Small peptides as mimetics of the protein hormone erythropoietin Science 1996, 273 (5274) 458-64 und Cwirla S. E. et al. Peptide agonist of the thrombopoeitin receptor as potent as the natural cytokine, Science 1997, 27653191696-9 und Qureshi S.A. et al. Mimicry of Erythropoietin by non peptid molecules, Proceedings National Academy of Sciences PNAS USA 1999, (9621): 12156-61). Die Bindung von EMP 1 an den Rezeptor geschieht an Bindungsstellen, die homolog zu den Hotspots der Wachstumshormonrezeptorverbindungen sind. Die Struktursequenzen von Thrombopoietin sind im Detail in EP 1 201 246 A2 beschrieben. Die Struktursequenzen von Erythropoietin sind im Detail in der europäischen Patentanmeldung EP 84 308 654.7 beschrieben.

In einer besonders vorteilhaften Ausführungsform wird EPO verwendet, das *in vivo* die Bildung von Erythrozyten sowie die Zellteilung und Differenzierung von Erythrozyten-Vorläuferzellen stimuliert. EPO ist entweder aus Urin isolierbar oder aber rekombinant herstellbar. Die Herstellung eines rekombinanten humanen EPO ist Gegenstand der WO 86/03520. Des weiteren ist EPO Gegenstand der EP 0 148 605, EP 0 205 564, EP 0 209 539, EP 0 267 678 oder EP 0 411 678.

Es können jedoch auch Derivate des nativen oder rekombinanten EPO eingesetzt werden. So ist beispielsweise ein EPO-Derivat bekannt (EP 0 640 619 B1), das zusätzliche Glykosylierungsstellen und somit einen höheren Kohlenhydratanteil mit 22 Sialsäureresten aufweist. Der Vorteil dieser Modifikation besteht darin, daß mit einem erhöhten Anteil an Sisalsäure die Halbwertzeit des EPO im Plasma zunimmt, da nur ein nicht-sialisiertes EPO an dem am EPO-Abbau beteiligten Galactose-Rezeptor der Leber binden kann. Dieses EPO-Derivat - bekannt unter der Abkürzung NESP (*Novel Erythropoiesis Stimulating Protein* oder Darbepoetin) - weist zwar im Vergleich zum rekombinanten EPO eine an fünf Positionen veränderte Aminosäuresequenz auf. Es entspricht aber in seiner Wirkung auf die Stimulation der Erythrozytenbildung im wesentlichen dem nativen oder rekombinanten EPO (Fisher, J.W.: Erythropoietin: Physiology and Pharmacology Update. Erythropoietin 2003, 1-14). Die EP 0 640 619 B1 wird hinsichtlich der Struktur und Herstellung des NESP vollumfänglich in Bezug genommen.

Das NESP kann zur weiteren Verlängerung der *in vivo* Halbwertzeit vorteilhafterweise noch mit Polyethylenglykol (PEG) chemisch konjugiert werden, ohne daß damit eine Veränderung der biologischen Aktivität verbunden wäre. Ein derart modifiziertes NESP ist aus der WO 01/76640 bekannt, die hinsichtlich der Struktur und Bereitstellung dieses EPO-Derivats vollumfänglich in Bezug genommen wird.

Aus der WO 02/49673 A2 und WO 01/02017 A2 sind ebenso pegylierte Derivate des EPO bekannt, die neben der verlängerten Plasma-Halbwertzeit auch eine höhere klinische Wirksamkeit zeigen. Dazu werden beispielsweise durch gezielte Mutagenese 1 bis 6 zusätzliche Glykosylierungsstellen in die EPO-Aminosäuresequenz eingebracht. Auch dieses Derivat kann erfindungsgemäß eingesetzt werden.

Erfindungsgemäß kann EPO sowohl in ihrer humanen als auch in ihrer nicht-humanen Form eingesetzt werden.

Die hämatopoietischen Wachstumsfaktoren können vorteilhafterweise auch für die Kultivierung von Gewebekulturen *in vitro* eingesetzt werden. Dazu werden die Zellen in für das Gewebe besonders geeigneten Vorrichtungen und Verfahren kultiviert, beispielsweise auf einem Gitter mit einer schneidenden Maschenstruktur von 500 m Größe, um immer wieder neue Tochteraggregate von Hepatozyten entstehen zu lassen. Insbesondere in vitro sind Kombinationen von EPO mit GH vorteilhaft.

Insbesondere können berührungslose, automatisch oder manuell gesteuerte Pumpsysteme eingesetzt werden, die z. B. aus Kolbenpumpen bestehen oder magnetisch bzw. durch Druckluftkompression von Schläuchen erzeugte, gerichtete Ströme erzeugen. In Anwesenheit von Endothelzellen kann es durch den Scherstress in einem perfundierten Bioreaktor zu einer spontanen Konfluenz der Endothelzellen auf den Oberflächen der Aggregate kommen, was für die weitere Verwendung vorteilhaft sein kann.

Für die Einkapselung eignen sich dem Fachmann bekannte, geeignete Materialien, in die beispielsweise strukturierte Formen oder Räume integriert werden, die eine *in situ* Wachstumsstruktur bzw. Vergrößerung ermöglichen. Alternativ kann auf die Kapsel verzichtet werden und beispielsweise in Gegenwart von Endothelzellen eine Endothelialisierung und somit eine optimale Hämokompatibilität erreicht werden.

Die Gabe von EPO führt entweder alleine oder bei grösseren strutkurellen Defekte auch in Verbindung mit scaffold Materialien zu einem biologischen Gewebeersatz. Diese Trägermaterialien können in vitro bzw. extrakorporal vorbesiedelt sein, biologisch modellierbar (biodegradabel) sein und sind mikro- und makrostrukturell und biochemisch im Idealfall der zu ersetzenden Struktur möglichst ähnlich. Die biochemische Nähe bzw. Identität beinhaltet eine Rekonstruktion der in vivo Zusammensetzung durch Kollagene und Matrixproteine (Elastin, Fibronektin und alle Matrixkomponenten des Körpers in gewebespezifischer Prägung wie bekannt).

Stammzellen können aus den verschiedenen im Körper des Patienten vorhandenen Quelle erhalten werden: Knochenmark, peripheres Blut, Fettgewebe, dem Gewebe selbst, Nabelschnurblut- bzw. Gewebe. Allogene Stammzellen können entsprechend gewonnen werden, sind aber mit immunologischen Nachteilen behaftet. Nichthumaneembryonale Zellen können verwendet werden, sind aber mit den entsprechenden Nachteilen behaftet.

Das Verfahren eignet sich insbesondere für adulte Zellen, d. h. primär differenzierte Zellen, die keinen embryonalen oder fötalen Phänotyp mehr besitzen. Es eignet sich insbesondere für humane adulte Zellen, beispielsweise für adulte Progenitorzellen, gewebespezifische Zellen, vorzugsweise Hepatozyten.

Es ist besonders vorteilhäft, die efindungsgemäße Verwendung lokal *in vivo* einzusetzen. Dazu können die Wachstumsfaktoren z.B. auf die Resektionsfläche eines Organs (Leber) aufgebracht werden. Sie können topisch oder aber über mit Hilfe eines Katheters lokal oder systemisch appliziert werden. Im Falle einer Leberresektion können sie alternativ oder ergänzend vor, während oder nach dem Eingriff appliziert werden. Ebenso ist es möglich, die Wachstumsfaktoren (z.B. zur Förderung der Knorpelregeneration) unmittelbar in das betroffene Gewebe oder Gelenk zu injizieren. Damit können die Wachstumsfaktoren über die Synovialflüssigkeit direkt auf die Bildung einer neuen Knorpelstruktur wirken.

In einer weiteren Ausführungsform können zusätzlich zu den hämatopoietischen Wachstumsfaktoren einer oder mehrerer der folgenden Wachstumsfaktoren verabreicht werden: "Transforming Growth Factor beta" (TGF beta), Prostaglandine, Granulozyten (-Makrophagen)-stimulierender Faktor (G(M)-CSF), "Growth Hormone Releasing Hormone" (GHRH), "Thyrotropin-releasing Homone" (TRH), "Gonadotropin-releasing Hormone" (GnRH), "Corticotropinreleasing Hormone" (CRH), Dopamin, "Antidiuretic Hormon" (ADH), Oxytocin, Prolactin, Adrenocorticotropin, beta-Celltropin, Lutrotropin und/oder Vasopressin verwendet bzw. zusätzlich ein oder mehrere Nervenregenerationsfaktoren, vorzugsweise "nerve growth factor" (NGF) und/oder ein oder mehrere Gefäßregenerationsfaktoren, vorzugsweise "Vascular Endothelial Growth Factor" (VEGF) und/oder "Plateled Derived Growth Factor" (PDGF).

Die hämatopoietischen Wachstumsfaktoren parenteral als injezierbare Mikrosphären, erodierbare Partikel, Polymerverbindungen (mit Polypeptid, Polyglycolsäure), Liposomen und kleine Partikel appliziert werden. Injezierbare oder implantierbare Drug Delivery -Geräte sind ebenso möglich. Die Stoffe können auch inhaliert werden, subkutan, intramuskulär gespritzt werden oder - für die topische Applikation - als kutanes Pflaster gegeben werden.

Es ist bekannt, dass aus Proteinen Mischpräparationen vorbereitet werden können. Suspensionen, Gelimulsionen, Feststoffverbindungen, dehydrierte oder lyophilisierte Puder sind möglich. Die Wachstumsfaktoren können auf Partikeln absorbiert werden oder enkapsuliert werden.

Es kann besonders vorteilhaft sein, Stammzellen (Progenitoren im eigentlichen Sinne) gemeinsam mit EPO zur Geweberegeneration einzusetzen, um damit das Recruitment der Gewebezellen aus den 4 Grundgewebearten für den Regenerationsprozess deutlich zu beschleunigen. EPO sowie die anderen genannten Faktoren kann gemischt mit Stammzellen und z.B. Fibrinkleber als Trägermatrix appliziert werden. Bei Bedarf kann die Trägermatrix weggelassen werden bzw. durch eine stärker vorstrukturierte und geformte Trägermatrix ersetzt werden. Die Faktoren können auch ohne jegliche biologische Trägermatrix z.B nur in wässrigrer Suspension systemisch oder topisch verabreicht werden.

Die erfindungsgemäße Gabe von EPO verbessert diese Geweberegeneration durch gewebespezifische Prägung der Stammzellen und Differenzierung im Anschluss an eine Vermehrung und Integration und koordiniert das Wachstum in Bezug auf die Grundgewebearten.

### I. Anwendungsbereiche

### Leber

Nach viralen Infektionen der Leber (z.B. Hepatitis A, B, C, D) kann es zu Regenerationsdefekten der Leber kommen. Die Lebercirrhose stellt das Endstadium eines Regenerationsdefektes der Leber dar, bei der es zu bindegewebigem Ersatz der parenchymatösen Zellen gekommen ist, da die körpereigene Regeneration nicht entsprechend mit der Rate einer durch Noxen bedingten Schädigung durch kontinuierliche Regeneration mithalten kann.

Nach Trauma und operativen Eingriffen, z.B. bei Tumorresektionen der Leber, kommt es zu einem akuten Verlust viablen Gewebes und zur Gewebezerstörung.

Erfindungsgemäß kommt es nach Gabe von EPO zur beschleunigten Regeneration der Hepatozyten und nicht-parenchymalen Zellen der Leber bis zur Restitutio ad Integrum. Charakteristisch hierfür ist die Wiederausbildung einer normalen Leberläppchen Architektur, die Wiederausbildung des interlobulären Bindegewebes, der Zentralvenen und der Glisson-Trias: Dort werden erkennbar die Gallengänge, Lymphgefäße, periportale Bindegewebe, Arterien und Vena interlobularis neben den Leberzellen wiederhergestellt.

Die spezifische Ausbildung der intralobulären Retikulinfasergeflechts und der Lebersinusoide wird wieder hergestellt. Der histologische Aufbau nach Regeneration zeigt die typische Balkenstruktur. Die Fensterungen im Bereich der Endothelzellen weisen auf eine typische Leberausprägung hin. Wichtige Initiatorzellen nach Trauma sind die Kupfferzellen die IL-6, IL-1, TNF-alpha freisetzen.

Die Gabe von EPO führt zu deutlich höheren Regenerationsprozessen selbst im Vergleich zu Lebergewebe ontogenetisch junger Lebern. In vitro-Faktorerhöhung um Faktor 10 bis 30 werden erreicht.

### II. Ausführungsbeispiele

### 1. Zelltransplantation

### Hepatozyten:

Leberzellen werden durch Collagenase-Verdau in üblicher Weise aus nicht tranplantierbaren Organen bzw. Leberresektaten isoliert. (Bader, A., Rinkes, I.H.B., Closs, I.E., Ryan, C.M., Toner, M., Cunningham, J.M., Tompkins, G.R., Yarmush, M.L. (1992) A stable long-term hepatocyte culture system for studies of physiologic processes: Cytokine stimulation of the acute phase response in rat and human hepatocytes. Biotechnol Prog. 8, 219-225.)

Die isolierten bzw. vorkultivierten Zellen werden in flüssigem Stickstoff gelagert. Nach dem Auftauen der Zellen entsprechend bekannter Protokolle (Karim, N., Allmeling, C., Hengstler, J.-G., Haverich, A., Bader, A. (2000) Diazepam metabolism and albumin secretion of porcine hepatocytes in collagen-sandwich after cryopreservation. Biotechnol Letters 22: 1647-1652) werden die Hepatozyten Suspension / Kulturen mit 100-150 IU/kg/KGW Epoetin alfa (bezogen auf den Empfänger) versetzt. Epoetin alfa wird hierzu in einer sterilen Lösung in einem Volumen von 1 - 1.5 ml einer 10 ml Hepatozytensuspension mit einer Konzentration von 2-10 Millionen / ml Zellen zugegeben.

Diese Suspension wird langsam (1 ml / Minute) intraportal injiziert. Die Suspension kann zusätzlich mit 1000 IE Heparin versetzt werden, um eine Thrombosierung zu vermeiden.

Alternativ kann das Zell / EPO Gemisch auch unter die Leberkapsel oder direkt in das Leberparenchym an mehreren Stellen injiziert werden. Hierzu empfiehlt es sich die Konzentration der Hepatozyten um den Faktor 2-5 zu erhöhen und das injizierte Volumen entsprechend zu reduzieren.

Die Stichkanäle werden mit handelsüblichen Fibrinkleber (Baxter Tissucol) verschlossen. Alternativ kann eine Tamponade mit Kollagenflies verwendet werden. Die Tamponade kann ebenso mit der Epoetin alfa Lösung versetzt werden. Es ist darauf zu achten, dass die Tamponade an der Adhäsionsstelle noch trocken bleibt.

Der Fibrinkleber stellt ein 2 Komponentenmix dar. Üblicherweise besteht eine Komponente aus Fibrinogen und die andere aus einer Aktivierungslösung mit Ca⁺⁺ und Proteinaseinhibitoren (z.B. Aprotinin). In die Aktivierungslösung kann Epoetin alfa durch Hinzumischen auf eine Endkonzentration von 100-150 IU/kg/KGW gegeben werden.

In analoger Weise können Stammzellen aus dem Knochenmark, dem Fettgewebe, einem spezifischen Parenchym oder dem Blut (Aufreinigung aus Buffy Coats, CD 34 positive Zellen) aus Nabelschnurblut und den mesenchymalen Zellen aus dem Nableschnurgewebe verwendet werden.

Parallel zur Zelltransplantation in ischämisch, toxisch, infektiös oder mechanisch (traumatisch) geschädigte Bereiche können die Zellen in einen Fibrinkleber oder autologes Plasma eingebracht und unter Zusatz von Epoetin alfa (100-150 IU/kg/KGW) zur Polymerisation gebracht werden.

Parallel dazu kann eine EPO-Gabe von jeweils 10000 IE s.c. begonnen werden, so dass insgesamt 40000 IE innerhalb einer Woche appliziert werden.

Das Ergebnis ist eine um den Faktor 2-3 erhöhte Geweberegeneration, die zu einer strukturellen Reparatur führt.

### 2. Postoperative Gabe

Epoetin alfa wird hier in einer Konzentration von 100-150 IU/kg/KGW gegeben.

Durch die EPO Gabe kommt es zu einer endogenen Erhöhung des Wachstumshormons um den Faktor 2, wodurch die Geweberegeneration nach der Operation beschleunigt wird. Die restitutio ad integrum tritt um ca. 1-2 Wochen früher als bei vergleichsweise ohne EPO Gabe behandelten Patienten ein.

EPO kann auch nach einer split liver Transplantation bzw. Lebertranplantation zur Induzierung der Leberregeneration gegeben werden.

Nach einer Lebertransplantation kommt es vor, dass das transplantierte Gewebe - oder bei Lebendspendem - das verbleibende Gewebe nicht rasch genug und in ausreichender Masse als aktives Gewebe zur Verfügung steht. In diesem Fall kann bereits 24 h vor der Operation und zum Zeitpunkt der Operation und danach in 24 h Abständen 100-150 IU/kg/KGW Epoetin alfa gegeben werden. Hierdurch kommt es zu einer deutlich beschleunigten Leberregeneration wobei postoperativ insbesondere am 4-5 Tage eine Volumenzunahme im Ultraschall diagnostizierbar ist.

### 3. Gabe nach operativer Leberresektion bei gut - und bösartigen Tumoren

Bei einer ausgedehnten Leberresektion besteht der Bedarf, eine beschleunigte Regeneration zu erreichen, da die Verfügbarkeit einer ausreichenden Zellmasse wichtig für das Überleben der Patienten ist. Nach operativer Resektion kann bei systemischer Gabe 100-150 IU/kg/KGW Epoetin alfa oder einer entsprechenden Menge bei topischer Applikation (in Fibrinkleber, Plasma) gegeben werden.

Bei Verdacht auf persistierenden Tumorbefall bzw. nicht ressezierbaren Tumormetastasten können sowohl systemisch als auch topisch entsprechend dem Tumortyp und der Prognose übliche Cytostatika in Kombination mit EPO verabreicht werden.

Nach Resektion und Gabe von EPO kommt es zu einem beschleunigten Grössenwachstum der behandelten Gruppe um 30% im Verhältnis zu einer nichtbehandelten Gruppe. Vor allem die Hepatozyten an den Resektionsflächen treten verstärkt in den strukturellen Wachstumsprozess ein. Es kommt zu einer vollständigen Ausbildung des Gefäßbaumes und des darum befindlichen Gewebes. Hierbei sind die Hepatozyten in typischer dem normalen Organ entsprechender Weise in Lobuli mit Gefäßversorgung, Zellplatten mit nichtparenchymalen Zellen (Kupffer, Pit, Ito - und Endothelzellen) angeordnet.

Es kommt zu einem systemischen Grössenwachstum. Das Größenwachstum endet bei Erreichen der initialen Grösse.

Die Gruppe mit perioperativer EPO-Gabe weist bereits am 1-2 Tag postoperativ einen um etwa 0.5 g/dl höheren Hb-Wert auf. Dies ist als Zeichen der bekannten Wirkung des EPO zu werten. Parallel kommt es jedoch zur Leberregneration. Hier vermehren sich nicht nur die Hepatozyten sondern alle Zelltypen und vor allem auch die Bindegewebsstrukturen, die das Architekturgerüst der Leber darstellen.

### Versuchsdurchführung:

28 weibliche Schweine (Gewicht 40,0 - 62,0 kg) wurden nach dem Zufallsprinzip in drei Gruppen aufgeteilt. Die Teilentfernung der linksseitigen Leber wurde mit der Technik der Bauchendoskopie durchgeführt.

Der Kontgruppe (n=16) wurde kein EPO gegeben. Der Gruppe 2 (n=6) wurde eine Kombination aus 10.000 Einheiten EPO und einem Fibrin-Dichtungsmittel (Quixil) lokal auf die Leberresektionsoberfläche gegeben. Gruppe 3 wurde gleichermaßen behandelt; die Schweine erhielten jedoch zusätzlich zur lokalen EPO Behandlung 10.000 Einheiten EPO systemisch am Tag 0, 3, 7 und 11.

Leberproben wurden von dem herausgeschnittenen Leberstück am Tag 0, 24 Stunden nach Resektion aus einem Bereich 1 cm unterhalb der Resektionsoberfläche und 14 Tage nach Resektion unterhalb der Resektionsoberfläche und des rechten seitlichen Lappens entnommen.

Für den Nachweis von Ki-67 Antigen, PCNA (proliferating cell nuclear antigen = PZNA Proliferation Zellkern Antigen) und Apoptose, wurde der Streptavidin-Biotin Immunperoxidase Assay an mit Formalin fixierten und in Paraffinwachs eingebetteten Lebergewebe durchgeführt.

### Ergebnisse

| | | Gruppe 1 (Kontrolle n=16) | Gruppe 2 (Wachstumsfaktor lokal) | Gruppe 3 (Wachstumsfaktor lokal und systemisch) |
|---|---|---|---|---|
| Lebervolumen (ml) | | 892,071 ± 130,56 | 894,02 ± 104,705 | **1073,10 ± 190,13*** |
| Lebergewicht (g) | | 1001,55 ± 155,76 | 1027,18 ± 166,95 | **1249,42 ± 222,51*** |
| Hb(mmol/l) (14. Tag) | | 6,0077 ± 0,65 | 6,550 ± 0,89 | 6,58 ± 0,5541 |
| Gewicht des Schweins (kg) | | 48,37 ± 5,25 | 52,67 ± 6,76 | 50,54 ± 9,801 |
| | | | | |

| | Gruppe 1 Kontrollgruppe | | Gruppe 2 Wachstumsfaktor lokal | Gruppe 3 Wachstumsfaktor lokal und systemisch |
|---|---|---|---|---|
| Ki 67 14. Tag (%) | 1,85 ± 2,01 | | **14,0 ± 12,11*** | **15,08 ± 15, 71*** |
| PZNA (%) | 25,33 ± 9,82 | | 29,87 ± 7,18 | 37,16 ± 14,32 |
| Apoptose (%) | 0,56 ± 0,429 | | 0,267 ± 0,103 | 0,56 ± 0,30 |

| | | | | |
|---|---|---|---|---|
| Arithmetisches Mittel und Standardabweichung * p ≤ 0,05 | | | | |

### 4. Optimierte Endoprothese bzw. Implantate (Vergleichsbeispiel)

Implantate können aus biologisch abbaubaren aber auch aus permanenten Materialien bestehen. Ein Beispiel hierfür sind metallische Endoprothesen z.B. im Hüftbereich. Im Anschluss an die Herstellung der metallischen Rohform wird eine Mikrostrukturieurng durch Abrasion, Ätzen oder Laserbehandlung erreicht. Hierdurch können offene Porositäten und Rauhigkeiten im Bereich von 1 bis 50-60 µm erzeugt werden.

Diese Strukturen werden anschließend in einer Lösung aus phasenreinem beta Tricalziumphosphat aufgefüllt, so dass eine homogene Oberflächenbeschichtung erreicht wird. Danach werden die Strukturen idealerweise einem weitergehendem Sinterungsprozess unterzogen, um die beta-TCP Strukturen zu festigen.

In den mineralischen Strukturen können lösliche Salze / Zucker eingebaut werden, bzw. Gasbildungen induziert werden um weitere interkonnektierende Porositäten zu erreichen. Im Anschluss an diesen Fertigungsprozess werden die Strukturen mit dem erfindungsgemäßen Wachstumsfaktor oder dessen Derivaten, Teilen oder Analoga imprägniert bzw. beschichtet. Depots können entsprechend an den Oberflächen gesetzt werden, indem die Kavitäten sich mit EPO füllen bzw. slow release Substanzen verwendet werden. Alternativ kann auch unmittelbar vor der Implantation das steril aus der Verpackung entnommen Implantat mit EPO und deren Analoga beschichtet werden.

Es kommt dadurch zu einem verbesserten Gewebeverbindungsprozess mit dem Implantat. Der Knochen wird makrovaskulär verbunden und dass Implantat beschleunigt und nachhaltiger ossär integriert.

In ähnlicher Form können Implantate im Mund-, Kiefer - und Gesichtsbereich vorbereitet werden (Zahnimplantate).

Eine Verbindung mit einer zellulären Besiedlung in Bioreaktoren mit Stammzellen ist möglich.

Biologische Implantate (Gefäße, Herzklappen, Haut) und Membranen können ebenso mit EPO und oder GH und oder TPO beschichtet werden.

### 5. Behandlung der Osteoporose (Vergleichsbeispiel)

EPO beschichtete Tricalziumphosphat Granula werden mittels einer Nadelpunktion in defizitäre / rarefizierte Wirbelkörper in einer autologen Plasmalösung eingebracht. Dort werden die Granula beschleunigt in endogenen Knochen umgebaut und der Degenerationsprozess in einen anabolen Effekt umgewandelt.

Der Effekt kann bei akut einbruchgefährdeten Wirbelkörper verwendet werden. Eine Kombination mit einem Besiedlungsprozess vorteilhafterweise mit Stammzellen aus dem autologen Knochenmark bzw. dem Periost und Blut und / oder Fettgewebe.

### 6. Indikation Knorpelregeneration (Vergleichsbeispiel)

Knorpelzellen stellen stark bradytrophe Gewebe dar. Durch ein regionales Trauma und Abrasion entstehen Entzündungsprozesse, die in eine Arthitis münden können. Durch Gabe von EPO in den Gelenkspalt bzw. systemisch und / oder in Kombination mit zellulären Transplantaten von Chondrozyten bzw. osteochonralen Zylindern wird die Geweberegeneration und der strukturelle Neuaufbau gefördert. Die kombinierte systemische bzw. subkkutane Gabe von 10000 IE / Tag ist möglich.

### 7. Indikation Hauterkrankungen (Vergleichsbeispiel)

Schlecht heilende Wunden werden nach Vorbereitung des Wundbettes erfindungsgemäß mit EPO oder TPO (Derivate, Analoga, Teile) beschichtet. Hierzu wird bevorzugt ein mechanisches Debridement durchgeführt. In die Blutkoagel werden 10 000 IE EPO eingebracht. Diese Prozess kann bis zur Reinigung des Wundgrundes wiederholt werden.

Das strukturelle Wachstum beginnt nach 2-3 Tagen führt zur beschleunigten Ausbildung eines Granulaitionsgewebes.

### 8. Indikation entzündliche Darmerkrankungen (Vergleichsbeispiel)

Bei Entzündungsphänomenen des Darmtraktes mit Gewichtsverlust und Anämie hat sich herausgestellt dass die Anämie nicht ein Sekundärphänomen auf Grund der schlechten Nahrungsresorption ist, sondern Begleiterscheinung eines originären EPO Mangels sein kann. Die Gabe von EPO in 10 000 IE / Tag führt zu einer Verbesserung der Darmwiederherstellung / Regeneration.

### 9. Indikation Neuroregeneration (Vergleichsbeispiel)

Nach Druchtrennungen des Rückenmarks führt EPO zu einem strukturellem Wachstum der Neurone und Neuaussprossung der Axone. Unterstützend wirkt die Gabe von Vitamin C.

EPO kann regional in Verbindung mit Fibrinkleber / autologem Plasma und / oder körpereigenen Stammzellen (Knochenmark, Blut CD 34, aus Fettzellen, Periost, Nabelschnur) gegeben werden.

### 10. Indikation Parkinson / Beispiel einer chronischen Erkrankung mit bereits abgeklungener Entzündungsreaktion (Vergleichsbeispiel)

Autologe Makrophagen, die durch Stimulation mit degradablen Partikeln stimuliert wurden, werden stereotaktisch in degenerierte Areale integriert. Parallel hierzu wird EPO (10 000 IE) in Verbindung mit autologen Stammzellen (0.3 ml) in das Areal injeziert. Parallel hierzu wird EPO über 2 Wochen stemisch verabreicht. Dieses Stimulationsprinzip der Induzierung einer Entzündung durch Makrophagen kann auch bei anderen chronischen Erkrankungen eingesetzt werden, bei denen kein akutes Trauma oder eine akute Entzündungsreaktion vorherrscht.

### 11. Wundheilung nach Brandverletzung (Vergleichsbeispiel)

Bei 8 brandverletzten Patienten heilten bei EPO Gabe die Donorstelle des Hauttransplantates um 50% schneller ab. Tief zweitgradige (Grad 2B) Verbrennungswunden im Gesicht heilten ohne Narbenbildung ab, wenn EPO den Patienten gegeben wurde. Ohne EPO Gabe heilten derartige Wunden mit Narbenbildung ab.

## Patentansprüche

1. Verwendung von Erythropoietin (EPO) zur Herstellung eines Medikaments zur Förderung der beschleunigten strukturellen Regeneration von Lebergewebe nach erfolgter operativer Leberresektion bei gut- und bösartigen Tumoren, wobei die Regeneration die Ausbildung eines Gefäßbaumes um die Resektionsfläche herum, Wiederausbildung einer normalen Leberläppchen-Architektur, des interlobulären Bindegewebes, der Zentralvenen und der Glisson-Trias sowie Erreichen der initialen Größe der Leber umfasst.

2. Verwendung von EPO nach Anspruch 1, wobei das Medikament zur topischen oder systemischen Anwendung vorgesehen ist.

3. Verwendung nach Anspruch 1, **dadurch gekennzeichnet, dass** das Medikament in Kombinationsanwendung mit einem Cytostatika vorgesehen ist.

4. Erythropoietin (EPO) zur Verwendung in einem Verfahren zur beschleunigten strukturellen Regeneration von Lebergewebe nach erfolgter operativer Leberesektion bei gut- und bösartigen Tumoren, wobei die Regeneration die Ausbildung eines Gefäßbaumes um die Resektionsfläche herum, die Wiederausbildung einer normalen Leberläppchen-Architektur, des interlobulären Bindegewebes, der Zentralvenen und der Glisson-Trias sowie das Erreichen der initialen Größe der Leber umfasst.

5. EPO nach Anspruch 4 zur topischen oder systemischen Verwendung.

## Claims

1. Use of erythropoietin (EPO) for the preparation of a medicament for promoting accelerated structural regeneration of liver tissue after surgical liver resection in the case of benign and malignant tumours, where the regeneration comprises the formation of a vascular tree around the resection area, re-formation of a normal liver lobe architecture, interlobular connective tissue, central veins and Glisson's triads, and attainment of the initial size of the liver.

2. Use of EPO according to Claim 1, where the medicament is intended for topical or systemic use.

3. Use according to Claim 1, **characterized in that** the medicament is intended for combination use with a cytostatic agent.

4. Erythropoietin (EPO) for use in a method for accelerated structural regeneration of liver tissue after surgical liver resection in the case of benign and malignant tumours, where the regeneration comprises the formation of a vascular tree around the resection area, re-formation of a normal liver lobe architecture, interlobular connective tissue, central veins and Glisson's triads, and attainment of the initial size of the liver.

5. EPO according to Claim 4 for topical or systemic use.

## Revendications

1. Utilisation d'érythropoïétine (EPO) pour la préparation d'un médicament destiné à favoriser la régénération structurelle accélérée de tissu hépatique après résection hépatique chirurgicale dans le cas de tumeurs bénignes et malines, où la régénération comprend la formation d'un arbre vasculaire autour de la zone de résection, la reformation d'une architecture de lobe de foie normal, de tissu conjonctif interlobulaire, de veines centrales et de triades de Glisson, et l'obtention de la taille initiale du foie.

2. Utilisation d'EPO selon la revendication 1, dans laquelle le médicament est prévu pour une utilisation topique ou systémique.

3. Utilisation selon la revendication 1, **caractérisée en ce que** le médicament est prévu pour une utilisation en association avec un agent cytostatique.

4. Erythropoïétine (EPO) destinée à une utilisation dans une méthode de régénération structurelle accélérée de tissu hépatique après résection hépatique chirurgicale dans le cas de tumeurs bénignes et malines, où la régénération comprend la formation d'un arbre vasculaire autour de la zone de résection, la reformation d'une architecture de lobe de foie normal, de tissu conjonctif interlobulaire, de veines centrales et de triades de Glisson, et l'obtention de la taille initiale du foie.

5. EPO selon la revendication 4, pour une utilisation topique ou systémique.
